# EUROPEAN PATENT SPECIFICATION

(11) **EP 1 289 570 B1**
(45) Date of publication and mention of the grant of the patent: **10.09.2008**
(21) Application number: 01942205.4
(22) Date of filing: 15.06.2001
(51) Int. Cl.: A61K 51/00, A61K 51/12, A61K 9/127, A61K 103/00, A61K 103/40, A61P 35/00

(54) **LIPOSOMAL ENCAPSULATION OF ALPHA-EMITTERS AND USES THEREOF**
LIPOSOMALE EINKAPSELUNG VON ALPHASTRAHLERN, UND DEREN VERWENDUNG
ENCAPSULATION LIPOSOMALE D'EMETTEURS ALPHA ET UTILISATIONS

(30) Priority: 16.06.2000 US 212186 P
(43) Date of publication of application: 12.03.2003
(73) Proprietor: Sloan-Kettering Institute For Cancer Research, New York, New York 10021 (US)
(72) Inventor: SGOUROS, George, New York, NY 10021 (US); THOMAS, James Louis, New York, NY 10027 (US); SCHEINBERG, David, New York, NY 10025 (US); MCDEVITT, Michael R., Bronx, NY 10471 (US)
(74) Representative: Knowles, James Atherton
(86) International application number: PCT/US2001/019133
(87) International publication number: WO 2001/097859

(56) References cited:
- WO-A-01/60417
- US-A- 4 394 372
- US-A- 4 624 846
- US-A- 4 665 897
- US-A- 5 328 678
- US-A- 5 534 241
- US-A- 5 807 572
- US-A- 5 853 752
- US-A- 6 060 315
- CHOI H O ET AL: "Application of anion-exchange resin to remove lipophilic chelates from liposomes." ANALYTICAL BIOCHEMISTRY. JUL 1986, vol. 156, no. 1, July 1986 (1986-07), pages 176-181, XP008037340 ISSN: 0003-2697
- BARD D R ET AL: "Effect of the intra-articular injection of lutetium-177 in chelator liposomes on the progress of an experimental arthritis in rabbits" CLINICAL AND EXPERIMENTAL RHEUMATOLOGY 1985 ITALY, vol. 3, no. 3, 1985, pages 237-242, XP008037346
- HARRINGTON K J ET AL: "Biodistribution and pharmacokinetics of 111In-DTPA-labelled pegylated liposomes in a human tumour xenograft model: implications for novel targeting strategies." BRITISH JOURNAL OF CANCER. JUL 2000, vol. 83, no. 2, July 2000 (2000-07), pages 232-238, XP008037620 ISSN: 0007-0920
- DATABASE CAPLUS [Online] XP002948638 Database accession no. 2001:617871 & WO 01 60417 A2 23 August 2001
- KOSTARELOS K. ET AL: 'Liposome-mediated delivery of radionuclides to tumor models for cancer radiotherapy: a quantitative analysis' JOURNAL OF LIPOSOME RESEARCH vol. 9, no. 3, August 1999, PHILADELPHIA, PA, US, pages 407 - 424, XP000849464 ISSN: 0898-2104
- HWANG K.J. ET AL: 'Encapsulation, with high efficiency, of radioactive metal ions in liposomes' BIOCHIMICA ET BIOPHYSICA ACTA vol. 716, no. 1, 1982, pages 101 - 109, XP008066537

## Description

### BACKGROUND OF THE INVENTION

### Cross-reference to Related Application

This non-provisional patent application claims benefit of provisional patent application U.S. Serial number 60/212,186, filed June 16, 2000, now abandoned.

### Field of the Invention

The present invention relates generally to the field of radiotherapy. More specifically, the present invention relates to encapsulation of alpha particle-emitting radionuclides into multivesicular liposomes. Most specifically, the present invention relates to encapsulation of chelated actinium-225 into multivesicular liposomes and uses thereof.

### Description of the Related Art

Optimal treatment with many drugs requires maintenance of a drug level for an extended period of time. For example, optimal anti-cancer treatment with cell cycle-specific antimetabolites requires maintenance of a cytotoxic drug level for a prolonged period of time. The half-life of many drugs after an intravenous (IV), subcutaneous (SC), intraperitoneal (IP), intraarterial (IA), intramuscular (IM), intrathecal (IT), or epidural dose is very short, being in the range of a fraction of an hour to a few hours.

Liposomes are one potential targeting vehicle for drug delivery. Multivesicular liposomes (MVL), first reported by Kim, et al. (Biochim, Biophys. Acta, 728:339-348, 1983), are uniquely different from other lipid-based drug delivery systems such as unilamellar (Huang, Biochemistry, 8:334-352, 1969; Kim, et al., Biochim. Biophys. Acta, 646:1-10, 1981) and multilamellar (Bangham, et al., J Mol. Bio., 13:238-252, 1965) liposomes. In contrast to unilamellar liposomes (also known as unilamellar vesicles, or "ULV"), multilamellar and multivesicular liposomes (MVL) contain multiple aqueous chambers per particle. Because of the similarity in acronyms, multivesicular liposomes (MVL) are frequently confused with multilamellar liposomes (MLV). Nevertheless, the two entities are entirely distinct from each other. Whereas multilamellar liposomes (also known as multilamellar vesicles or MLV) contain multiple concentric chambers within each liposome particle, resembling the "layers of an onion," the multiple aqueous chambers in multivesicular liposomes are non-concentric. The structural differences between unilamellar, multilamellar, and multivesicular liposomes are well known in the art.

The structural and functional characteristics of multivesicular liposomes are not directly predictable from current knowledge of ULV and multilamellar liposomes. The differences are described on page 19 of the book Liposomes as Tools in Basic Research and Industry (Jean R. Philippot and Francis Schuber, eds., CRC press, Boca Raton, Fla., 1995, page 19). Multivesicular liposomes are bounded by an external bilayer membrane shell, but have a very distinctive internal morphology, which may arise as a result of the special method employed in the manufacture. Topologically, multivesicular liposomes (MVL) are defined as liposomes containing multiple non-concentric chambers within each liposome particle, resembling a "foam-like" matrix. The presence of internal membranes distributed as a network throughout multivesicular liposomes may serve to confer increased mechanical strength to the vesicle, while still maintaining a high volume:lipid ratio as compared to multivesicular liposomes. The multivesicular nature of multivesicular liposomes also indicates that, unlike for unilamellar vesicles, a single breach in the external membrane of a multivesicular liposome will not result in total release of the internal aqueous contents. Thus, both structurally and functionally the multivesicular liposomes are unusual, novel and distinct from all other types of liposomes. As a result, the functional properties of multivesicular liposomes are not predictable based on the prior art related to conventional liposomes such as unilamellar vesicles and multilamellar liposomes.

The prior art describes a number of techniques for producing unilamellar vesicles and multivesicular liposomes (for example, U.S. Pat. Nos. 4,522,803 to Lenk; 4,310,506 to Baldeschwieler; 4,235,871 to Papahadjopoulos; 4,224,179 to Schneider; 4,078,052 to Papahadjopoulos; 4,394,372 to Taylor; 4,308,166 to Marchetti; 4,485,054 to Mezei; and 4,508,703 to Redziniak). The prior art also describes methods for producing multivesicular liposomes (Kim, et al., Biochim. Biophys. Acta, 728:339-348, 1983). For a comprehensive review of various methods of unilamellar vesicles and multivesicular liposomes preparation, refer to Szoka, et al., Ann. Rev. Biophys. Bioeng., 9:465-508, 1980. In the method of Kim, et al. (Biochim. Biophys. Acta, 728:339-348, 1983), the pharmaceutical utility of multivesicular liposomes encapsulating small therapeutic molecules, such as cytosine arabinoside or cytarabine, is limited. Subsequent studies (Kim, et al., Cancer Treat. Rep., 71:705-711, 1987 and US5 807 572) showed that the release rate of encapsulated molecules into biological fluids can be modulated by encapsulating in the presence of a hydrochloride.

Kostarelos et al (Journal of Liposome Research 9(3), pp407-424, 1999) examined the potential clinical application of radionuclide conjugates with multilamellar vesicle, small unilamellar vesicle and sterically stabilised liposomes in internal radiotherapy.

WO 01/60417 published after the filing date of the present application discloses unilamellar and sterically stabilised liposomes for encapsulating heavy radionuclides emitting alpha particle for internal radiotherapy.

The prior art is deficient in an effective means for sequestering Ac-225 and its daughter radionuclides at specific targets during radiotherapy. The present invention fulfils this long standing need and desire in the art.

### SUMMARY OF THE INVENTION

Alpha particle-emitting radionuclides hold great promise as potential therapeutic agents for cancer treatment. In treating late-stage breast cancer patients (with measurable liver or bone metastases), long-lived alpha particle emitters are required to reach distant metastases that have developed their own vasculature. One of the most promising of such radionuclides, Ac-225 has a 10-day half-life and results in intermediates that yield a total of 4 alpha particles. This radionuclide is shown to be 1000-fold more effective than Bi-213, in animal studies, however, it has also proved to be far more toxic. The increased efficacy and toxicity are a result of the alpha-particle emitting intermediates. When these are confined to the target cells, efficacy is increased, when they distribute throughout the body, toxicity is increased. This is a fundamental difficulty if antibodies are to be used as the targeting vehicle since the bond holding the Ac-225 atom to the antibody will be broken after decay of Ac-225. This will leave the first daughter in the decay chain free to distribute throughout the body where it will decay and subsequently yield additional alpha emissions to normal organs from subsequent daughter decays. In short, of the 4 alphas, only the first one, originating from decay of Ac-225 contributes to the tumor dose, the remainder will distribute throughout normal tissue to increase toxicity. The present invention demonstrates that liposomal encapsulation of Ac-225 reduces loss of radioactive decay intermediates from the targeting vehicle and, therefore, the tumor site.

In one embodiment of the present invention, there is provided use of a composition in the manufacture of a medicament for a method for preventing the systemic release of radioactive decay intermediates upon administration of an alpha particle-emitting radionuclide to an individual. The radionuclide is incorporated into the aqueous phase of multivesicular liposomes so that the resulting radioactive decay intermediates remain sequestered within the multivesicular liposomes and are not systemically released into the individual. The alpha particle emitting radionuclide may be incorporated into the aqueous phase as a chelation compound. The instant invention is especially useful for the delivery of the alpha particle-emitting radionuclides ²²⁵Ac, ²²³ Ra, ²¹³ Bi and ²¹¹At, and utility of the instant invention for ²²⁵Ac delivery has been examined in depth.

In another embodiment, the use of the instant invention is used for the treatment of cancer.

In another embodiment of the instant invention, the liposomes such as defined in claim 1 are coated with molecules that preferentially associate with a target cell such as anti-tumor antibodies. As a result, both targeting and target retention of the liposomes are enhanced. One example of such a molecule would be an antibody.

In a further embodiment of the instant invention, retention of radioactive decay intermediates is facilitated by incorporating metal-binding or halogen binding molecules into the aqueous phase of the liposomes of claim 1.

In yet another embodiment of the present invention, non-tumor specific uptake of radionuclide containing liposomes of claim 1 into reticuloendothelial organs such as the spleen and liver is inhibited by preinjecting an individual with empty liposomes. to saturate the nonspecific absorption of liposomes into these organs.

Other and further aspects, features, and advantages of the present invention will be apparent from the following description of the embodiments of the invention given for the purpose of disclosure.

### BRIEF DESCRIPTION OF THE DRAWINGS

**Figure 1** shows the radioactivity collected in each fraction after Sephadex^{™} column chromatography at different times after ¹¹¹In encapsulation.
**Figure 2** shows the retention of ¹¹¹In in liposomes as a function of time after loading.
**Figure 3** shows the model of Ac-225 radioactive decay used to determine loss rate of daughter radionuclides from liposome-encapsulated Ac-225 activity.
**Figures 4A** and **4B** show sample simulations using models of the transfer rate from each sub-compartment within liposomes to the extraliposomal compartment.
**Figures 5A, 5B****,** **5C** and **5D** depict simulations obtained using 4 different loss rates.
**Figures 6A** and **6B** show the relationship between loss rate and the levels of daughter activity at different measurement times after liposome separation.

### DETAILED DESCRIPTION OF THE INVENTION

The present invention provides targeted delivery of alpha particle-emitting radionuclides and their alpha-emitting progeny as it relates, for example, to breast cancer therapy using liposome-encapsulated alpha emitters such as defined in claim 1.

More specifically, the instant invention provides a medicament for preventing the systemic release of radioactive decay intermediates upon administration of an alpha particle-emitting radionuclide to an individual by incorporating the radionuclide into the aqueous phase of multivesicular liposomes. The radioactive decay intermediates that remain sequestered within the multivesicular liposomes are not systemically released into the individual. The alpha particle emitting radionuclide may be incorporated into the aqueous phase as a chelation compound. The instant invention is especially useful for the delivery of the alpha particle-emitting radionuclides ²²⁵Ac_{,} ²²³Ra, ²¹³Bi and ²¹¹At. The utility of the instant invention has been especially evaluated for ²²⁵Ac.

The instant invention is especially directed to the use of the liposomes of claim 1 for the delivery of alpha particle emitting radionuclides for the treatment of cancer.

The present invention also provides a method in which the liposomes of claim 1 are coated with molecules that preferentially associate with a target cell. One example of such a molecule would be an antibody. Anti-tumor antibodies would be especially useful for targeting cancer cells.

The instant invention also includes a method to facilitate retention of radioactive decay intermediates by incorporating metal-binding or halogen binding molecules into the aqueous phase of the liposomes of claim 1.

The instant invention also provides a medicament to reduce non-tumor specific uptake of the radionuclide containing liposomes of claim 1 into reticuloendothelial organs such as the spleen an d liver by preinjecting empty liposomes into the individual to saturate absorption of liposomes of claim 1 into these organs.

The following examples are given for the purpose of illustrating various embodiments of the invention.

### Reference EXAMPLE 1

### Rationale for liposomal incorporation of alpha emitters

Alpha particle-emitting radionuclides hold great promise as potential therapeutic agents for cancer treatment. To date, only two such radionuclides, astatine-211 (At-211) and bismuth-213 (Bi-213) have been investigated clinically. Both have short half-lives (7 hours and 46 minutes, respectively) and are, therefore, appropriate for situations in which targeting is very rapid. Their use has been limited to leukemia patients, in which the disease is rapidly accessible by intravenous administration (Bi-213), or to patients that have undergone surgical excision of brain cancer, wherein the radionuclide was injected directly into the surgical cavity (Art-211). The former study demonstrated the low toxicity of IV-injected alpha emitters, while the latter approach has yielded prolonged survival in poor prognosis patients. In both studies, the radionuclide was attached to antibodies against tumor associated antigens. In treating other tumors, such as breast cancer with liver and bone metastases, longer-lived alpha emitters would be required to reach distant metastases which have developed their own vasculature. For such tumors, a more sophisticated targeting approach would be necessary.

Although liposomes have been used in the delivery of chemotherapy and in gene targeting, the use of liposomes in the delivery of radioactivity has not been accepted. This is primarily because high uptake of the liposomes was observed in reticuloendothelial organs, such as the liver and the spleen, during initial studies performed in the 1980's. Since that time, however, new liposomal systems have been generated with reduced reticuloendothelial uptake. Examples include sterically-stabilized liposomes coated with monosialogangliosides or polyethylene glycol (PEG). The use of such liposomes for the delivery of Ac-225, and other promising alpha emitters such as radium-223 (Ra-223), is particularly compelling because they may retain daughter radionuclide with the aqueous phase and thereby reduce systemic toxicity. Since the range of an alpha particle (50-100 microns) is sufficient to penetrate beyond the liposomal membranes (70nm), tumor irradiation will be enhanced.

### Reference EXAMPLE 2

### Analysis of indium-111 leakage from liposomes

To optimize the liposomal formulation and to evaluate potential leakage of radionuclides from liposomes constructs, indium-111 was used in place of Ac-225. Indium-111 is easily detected by gamma counting and served to develop and test the methodology for subsequent encapsulation of Ac-225.

Both Indium-111 and actinium-225 form tri-chloride complexes.

The method described by Hwang *et al.* was used with minor modifications. Liposomes were isolated by Sephadex^{™} chromatography and loaded with ¹¹¹In by incubation for 1 h at room temperature with a loading solution consisting of 6-10 µl 6.9 mM oxine in oxine sulfate in deionized water with 200 µl 1.8% NaCl/20mM sodium acetate, pH 5.5. To this acetate buffer, an equal volume of ¹¹¹InCl₃ in 3 mM HCl was added to make the final loading solution. Loading was terminated by passage through an AGIX ion-exchange column. The fractions corresponding to liposomes were pooled and stored at 4°C and 37°C for evaluation of indium leakage.

At different times after ion-exchange chromatography, aliquots of the pooled liposomal fractions were drawn, chromatographed by size exclusion (Sephadex^{™} column) separation and counted for radioactivity in a gamma counter. The counts in liposomal fractions were expressed as a fraction of the radioactivity aliquoted from the original pooled sample. In selected samples, prior incubation with DTPA was included to ensure that retention of ¹¹¹In in liposomal fractions was not the result of leakage and equilibration of ¹¹¹In between extra- and intra-liposomal ¹¹¹In in the pooled sample.

### Reference EXAMPLE 3

### Results from the indium-111 experiments

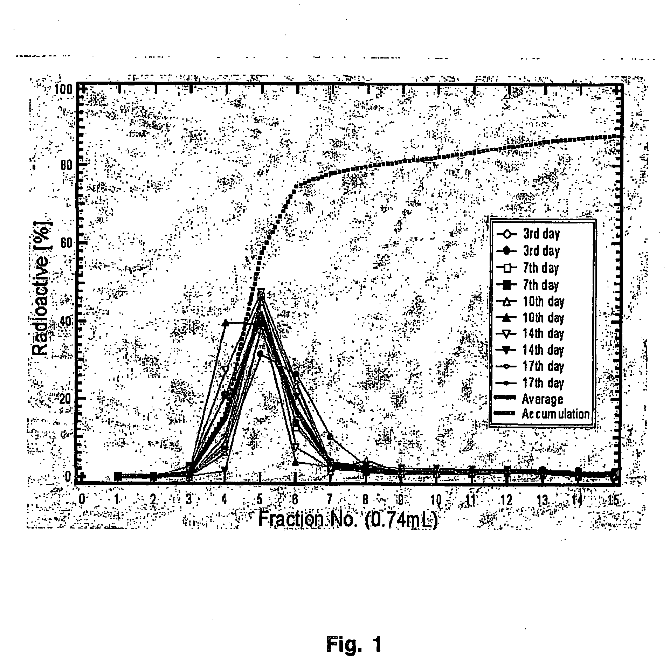

Following ion-exchange chromatography, approximately 20% of the radioactivity remains on the ion-exchange resin, and close to 80% in the liposomal fractions, yielding an encapsulation efficiency of approximately 80%. The radioactivity collected in each fraction after Sephadex^{™} column chromatography at different times after ¹¹¹In encapsulation is depicted in **Figure 1****.** The results show a generally time-invariant profile following size-exclusion separation.

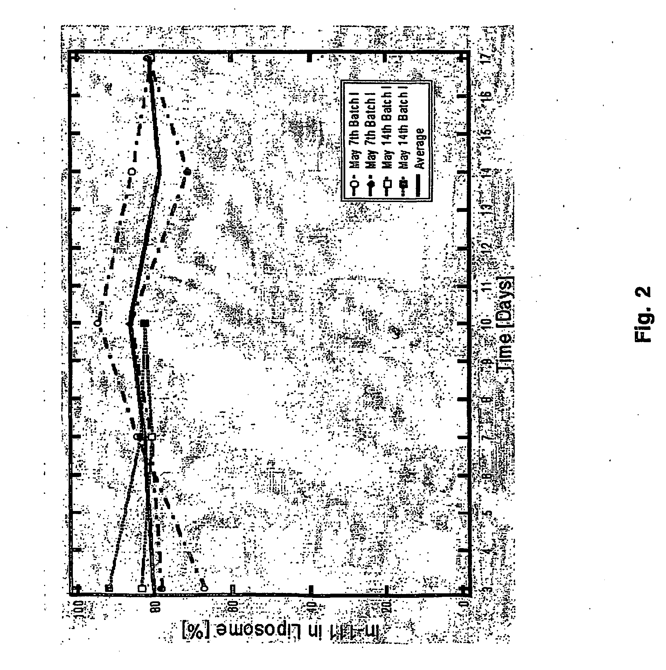

**Figure 2** depicts the retention of ¹¹¹In in liposomes as a function of time after loading. The fraction of ¹¹¹In retained within liposomes appears to remain constant at approximately 80% over a prolonged time-period, indicating that retention of Ac-225 within liposomes is possible.

### Reference EXAMPLE 4

### Analysis of actinium-225 leakage from liposomes

The next step in this work was to carry out these same experiments using Arc-225. In this case, gamma counting provided information regarding the presence of daughter radionuclides within the liposomes. Liposomes were incubated for 1 hr with Ac-225. To determine loss rate of daughter radionuclides from liposome-encapsulated Arc-225 activity, Ac-225 containing liposomes were separated from free Ac-225 by elution through a Sephadex^{™} column. The fractions corresponding to the liposomes (fractions 4-6, based on In-111 studies) were pooled and counted on a gamma counter using windows appropriate for detection of the Fr-221 and Bi-213 photopeaks (218 and 440 keV, respectively). A 1-minute counting interval was selected and counting was performed overnight Fr-221 counts were decay corrected to the start of the 1-min. counting period. At the end of separation and 10 to 20 h later (if possible), the column was also counted in a dose calibrator.

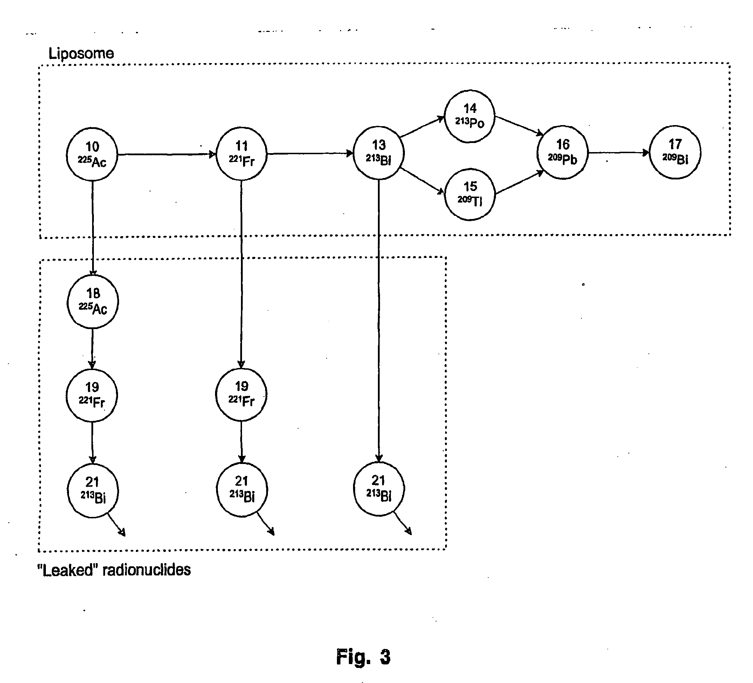

The results were analyzed using the model of Ac-225 radioactive decay shown in **Figure 3****.** Sub-compartments 10-17 correspond to decays that occur within liposomes. Loss of daughter (or parent) radionuclide is depicted by a "bleak" rate from the liposome compartments to corresponding sub-compartments in the "leaked" radionuclides compartment. The transfer rate from each sub-compartment within liposomes to the extraliposomal compartment reflects a rate of loss from liposomes This rate may be described in terms of a loss or clearance half-life, the amount of time required for Ω of a particular radionuclide to diffuse out of the liposome. Transfer rates within a compartment correspond to physical decay, whereas those that cross between the two compartments correspond to transfer of radionuclide from one to the other compartments. Since astatine-217, which has a 32 msec half-life, is not resolved by this model, it is lumped with Fr-221.

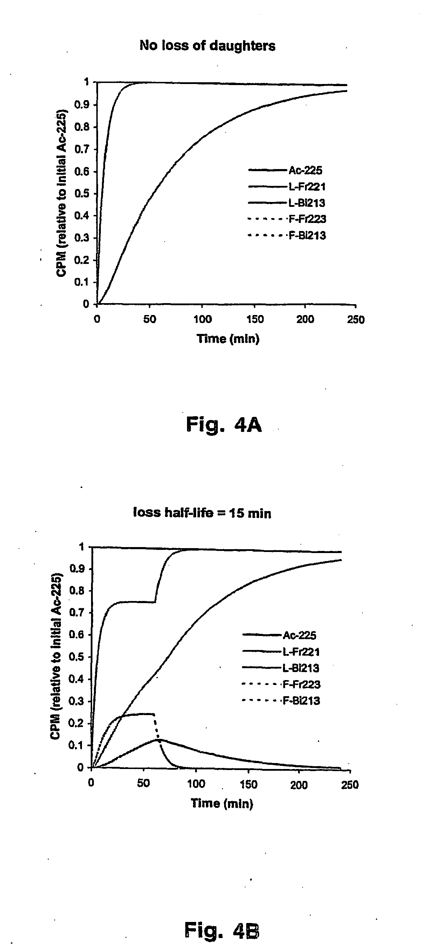

A sample simulation using this model is depicted in **Figures 4A** and **4B. Figure 4A** shows the results of a simulation in which complete retention of all radionuclides was assumed. Results are expressed relative to the initial activity of Ac-225 encapsulated by the liposomes. The first solid line shows the decay of Ac-225 while the other solid curves show the rise in daughter radioactivity within Liposomes as equilibrium between the parent and daughters is reached. Note that because of its short, 5 min half-life, Fr-221 achieved equilibrium much more rapidly than Bi-213, which has a 45.6 min half-life. The two dotted lines, corresponding to Fr-221 or Bi-213 activity not within liposomes, are just visible at zero throughout the simulation duration.

**Figure 4B** shows a simulation assuming loss of Fr-221 at a rate of 0.046 min⁻¹, which is equivalent to a loss half-life of 15 min. All other loss rates were set to zero. This means that Bi-213 generated within liposomes was assumed to remain there. Complete retention of Ac-225 was also assumed in this simulation.

As shown by the rise in both liposome-associated and free daughters (solid and dashed lines, respectively), equilibration and loss of daughters was allowed to occur during the 1-hr incubation period. After separation, and during counting of liposomal fractions, the distinction between free and liposome-encapsulated radioactivity was no longer possible. Therefore, the loss rate is turned off so that the solid curves correspond to total daughter radioactivity associated with the liposomal fractions (including daughters that were leaking out); the dashed curves reflect the physical decay of daughter radionuclides remaining in the column or in the liposome-free fractions. Although the latter could be assayed for radioactivity of Fr-221 and Bi-213, the method for assessing daughter loss rates was based upon counting the liposomal fractions.

### Reference EXAMPLE 5

### Analysis using different loss rates

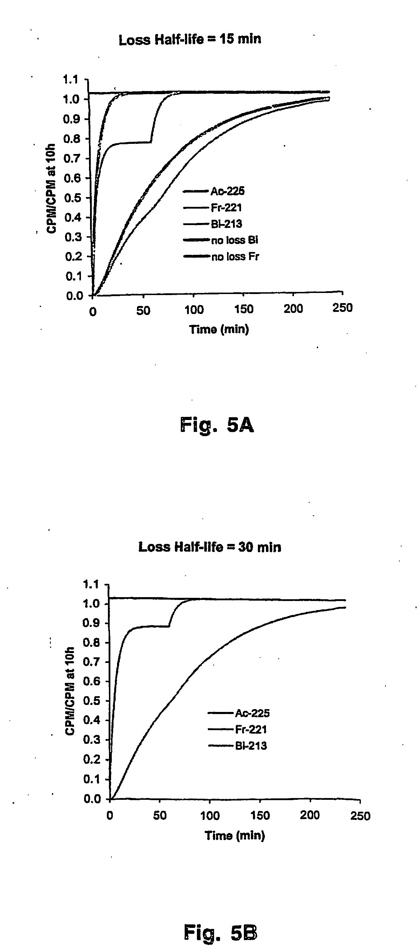

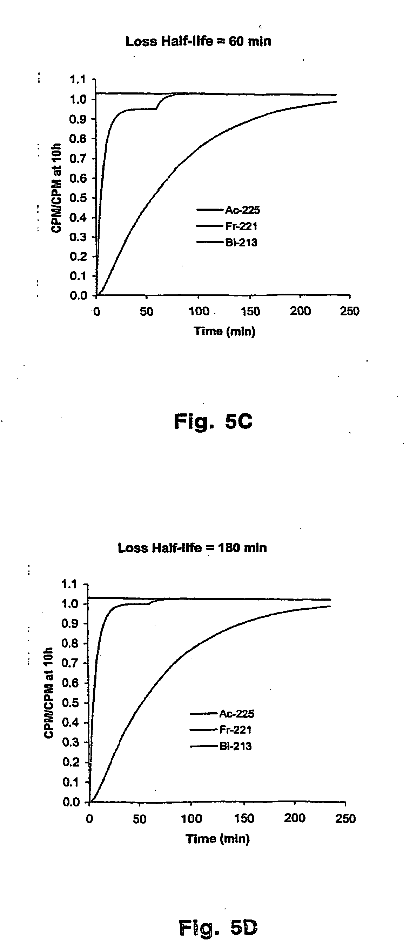

**Figures 5A-5D** depict simulations obtained using 4 different loss rates. The curves associated with counting of liposomal fractions are shown without the corresponding curves for free daughters collected on the column or in the liposome-free fractions. All other conditions are as described above. The CPM values are normalized by dividing by CPM expected after 10 h (i.e., at equilibrium).

In **Figure 5A****,** (loss half-life = 15 min) the curves corresponding to a simulation that did not include loss of Fr are shown for comparison with the simulation that included loss. The level of Fr-221 and Bi-213 activity immediately after separation was sensitive to the loss half-life of Fr-221. This is clearly evident for Fr-221 since the loss rate impacted the equilibrium level (plateau) that was reached. It was less evident for Bi-213 since it does not reach equilibrium in 1 h. Once the separation had occurred (i.e., after 60 minutes), the distinction between free and liposome-associated daughter activity was lost over time as the daughters reached equilibrium with the parent. Correspondingly, as the measurement of daughter activity was delayed relative to the time of separation, the ability to distinguish the different loss rates is also reduced.

### Reference EXAMPLE 6

### Relationship between loss rate and daughter activity

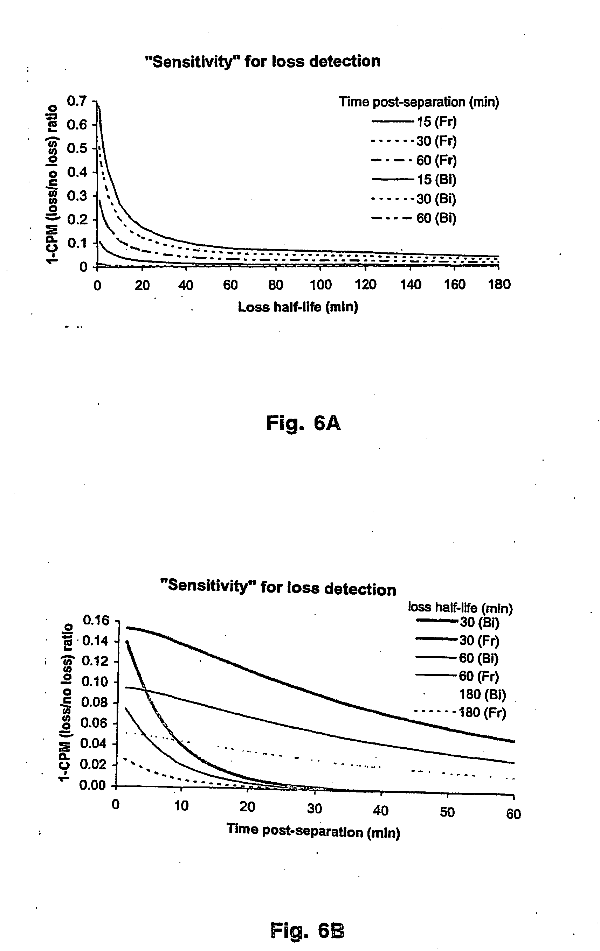

The relationship between loss rate and the levels of daughter activity at different measurement times after liposome separation is shown more directly in **Figure 6A-6B. Figure 6A** shows the normalized count rate ratio, as presented in **Figures 5A-5D****,** of each daughter divided by the corresponding count rate ratio assuming no loss of Fr-221 and then subtracted from one to yield curves that approach zero over time. Results are plotted against different loss half-lives and for measurement times of 15, 30 and 60 minutes after separation. If counting is started immediately after separation and is carried out overnight, these data are available.

**Figure 6B** is a different representation of the data used to generate **Figure 6A****.** Loss rate sensitivity is plotted against the time post-separation at which the liposomal fractions are counted. Curves are provided for three different loss half-lives: 30, 60 and 180 min.

### Reference EXAMPLE 7

### Determine the loss rate that yields a_negligible absorbed dose to kidneys from Bi-213

To determine the loss rate that yields a negligible absorbed dose to kidneys from Bi-213, a 5 day biological half-life of the Ac-225 construct is assumed and a 25 day simulation is plotted. Subsequently, the Bi-213 released for different Fr-221 release rates is integrated and is used to perform rough estimate of dosimetry. 25 day = 36000 min = simulation time 5 day half-life = 9.627e-5

### Reference EXAMPLE 8

### Possible enhancements of liposomal delivery of alpha emitters

Three further enhancements in the use of liposomes to deliver alpha emitters to tumors are also specifically contemplated within the context of the methods of the present invention. One is to incorporate metal-binding or halogen-binding molecules within the aqueous phase of the liposomes to bind and to enhance retention of daughter radionuclides. A second enhancement is the preinjection of large, empty liposomes to saturate the reticuloendothelial organs to reduce non-tumor specific spleen and liver uptake of radionuclides. The third and final approach is to coat the liposomes with anti-tumor antibodies to increase tumor localization and retention of the liposomes

The following references were cited herein:
Hwang, K. J., et al. "Encapsulation, with High Efficiency, of Radioactive Metal Ions in Liposomes." Biochim Biophys Acta 716.1 (1982): 101-9.

## Claims

1. The use of a composition comprising multivesicular liposomes and, incorporated into the aqueous phase of said multivesicular liposomes, an alpha particle-emitting radionuclide in the manufacture of a medicament for the treatment of an individual in need of radiotherapy, whereby radioactive decay intermediates formed by the radioactive decay of the radionuclide remain sequestered within said liposomes.

2. The use according to claim 1, wherein the alpha particle-emitting radionuclide is incorporated into the aqueous phase as a chelation compound.

3. The use according to either claim 1 or claim 2, wherein the alpha particle-emitting radionuclide is selected from ²²⁵Ac, ²²³Ra, ²¹³Bi and ²¹¹At.

4. The use according to claim 3, wherein the alpha particle-emitting radionuclide is ²²⁵Ac.

5. The use according to claim 1, wherein the individual in need of radiotherapy is being treated for cancer.

6. The use according to any one of claims 1 to 5, wherein the liposomes are coated with molecules which preferentially associate with a specific target cell to augment the targeting and target retention of said liposomes.

7. The use according to claim 6, wherein said molecules are antibodies.

8. The use according to claim 7, wherein said antibodies are anti-tumor antibodies.

9. The use according to any one of claims 1 to 8, wherein additional molecules are incorporated into the aqueous phase of said multivesicular liposomes to facilitate retention of radioactive decay intermediates, wherein said molecules are selected from metal-binding molecules and halogen-binding molecules.

10. The use according to any one of claims 1 to 9, wherein the medicament is for administration to the individual following the administration, by injection, of empty liposomes to saturate the reticuloendothelial organs to reduce non-tumor specific spleen and liver uptake of said radionuclide.

11. The use according to any one of claims 1 to 10, wherein auxiliary molecules are included in the liposome formulation in order to prevent uptake by the reticuloendothelial organs.

12. The use according to claim 11, wherein the auxiliary molecules are polyethyleneglycol-linked lipids (PEG-lipids).

13. The use according to claim 6, wherein the targeting molecules are attached to auxiliary molecules.

14. The use according to any one of claims 1 to 13, wherein additional molecules are incorporated into the formulation in order to facilitate membrane fusion with target cells or to facilitate endocytosis by target cells.

## Patentansprüche

1. Verwendung einer Zusammensetzung, die multivesikuläre Liposomen und, eingeschlossen in der wässrigen Phase von genannten multivesikulären Liposomen, ein Alpha-Teilchen emittierendes Radionuklid umfasst, bei der Herstellung eines Medikaments für die Behandlung eines Individuums, das Strahlentherapie benötigt, wodurch radioaktive Zerfallszwischenprodukte, die durch den radioaktiven Zerfall des Radionuklids gebildet werden, innerhalb von den genannten Liposomen abgekapselt bleiben.

2. Verwendung nach Anspruch 1, worin das Alpha-Teilchen emittierende Radionuklid in der wässrigen Phase als eine Chelatverbindung eingeschlossen ist.

3. Verwendung nach Anspruch 1 oder Anspruch 2, worin das Alpha-Teilchen emittierende Radionuklid aus ²²⁵Ac, ²²³Ra, ²¹³Si und ²¹¹At ausgewählt ist.

4. Verwendung nach Anspruch 3, worin das Zalpha-Teilchen emittierende Radionuklid ²²⁵Ac ist.

5. Verwendung nach Anspruch 1, worin bei dem Individuum, das Strahlentherapie benötigt, gegen Krebs behandelt wird.

6. Verwendung nach einem der Ansprüche 1 bis 5, worin die Liposomen mit Molekülen bedeckt werden, die sich vorzugsweise mit einer spezifischen Zielzelle assoziieren, um das Targeting (zielgerichteter Angriff) und die Zielretention von genannten Liposomen zu steigern.

7. Verwendung nach Anspruch 6, worin genannte Moleküle Antikörper sind.

8. Verwendung nach Anspruch 7, worin genannte Antikörper Anti-Tumor-Antikörper sind.

9. Verwendung nach einem der Ansprüche 1 bis 8, worin zusätzliche Moleküle in der wässrigen Phase von genannten multivesikulären Liposomen eingeschlossen sind, um die Retention von radioaktiven Zerfallszwischenprodukten zu begünstigen, worin genannte Moleküle aus Metallbindungs-Molekülen und Halogenbindungs-Molekülen ausgewählt sind.

10. Verwendung nach einem der Ansprüche 1 bis 9, worin das Medikament für die Verabreichung an ein Individuum nach der Verabreichung durch Injektion von leeren Liposomen vorgesehen ist, um die retikuloendothelialen Organe zu sättigen, um eine nicht-tumorspezifische Aufnahme von genanntem Radionuklid durch die Milz und Leber zu verringern.

11. Verwendung nach einem der Ansprüche 1 bis 10, worin Hilfsmoleküle in der Liposomformulierung eingeschlossen sind, um eine Aufnahme durch die retikuloendothelialen Organe zu verhindern.

12. Verwendung nach Anspruch 11, worin die Hilfsmoleküle Polyethylenglycol-verknüpfte Lipide (PEG-Lipide) sind.

13. Verwendung nach Anspruch 6, worin die abzielenden Moleküle an Hilfsmoleküle gebunden sind.

14. Verwendung nach einem der Ansprüche 1 bis 13, worin zusätzliche Moleküle in der Formulierung eingeschlossen sind, um Membranfusion mit Zielzellen zu begünstigen oder Endozytose durch Zielzellen zu begünstigen.

## Revendications

1. Utilisation d'une composition comprenant des liposomes multivésiculaires et un radionucléide émettant des particules alpha, incorporé dans la phase aqueuse desdits liposomes multivésiculaires, dans la fabrication d'un médicament pour le traitement d'un individu ayant besoin de radiothérapie, moyennant quoi les intermédiaires de décroissance radioactive formés par la décroissance radioactive du radionucléide demeurent séquestrés au sein desdits liposomes.

2. Utilisation selon la revendication 1, dans laquelle le radionucléide émettant des particules alpha est incorporé dans la phase aqueuse sous la forme d'un composé de chélation.

3. Utilisation selon la revendication 1 ou la revendication 2, dans laquelle le radionucléide émettant des particules alpha est sélectionné parmi ²²⁵Ac, ²²³Ra, ²¹³Bi et ²¹¹At.

4. Utilisation selon la revendication 3, dans laquelle le radionucléide émettant des particules alpha est ²²⁵Ac.

5. Utilisation selon la revendication 1, dans laquelle l'individu ayant besoin de radiothérapie est traité pour un cancer.

6. Utilisation selon l'une quelconque des revendications 1 à 5, dans laquelle les liposomes sont enduits de molécules qui s'associent de préférence à une cellule cible spécifique pour augmenter le ciblage et cibler la rétention desdits liposomes.

7. Utilisation selon la revendication 6, dans laquelle lesdites molécules sont des anticorps.

8. Utilisation selon la revendication 7, dans laquelle lesdits anticorps sont des anticorps anti-tumoraux.

9. Utilisation selon l'une quelconque des revendications 1 à 8, dans laquelle des molécules supplémentaires sont incorporées dans la phase aqueuse desdits liposomes multivésiculaires pour faciliter la rétention des intermédiaires de décroissance radioactive, dans laquelle lesdites molécules sont sélectionnées parmi des molécules se fixant à un métal et des molécules se fixant à un halogène.

10. Utilisation selon l'une quelconque des revendications 1 à 9, dans laquelle le médicament est pour l'administration à l'individu suivant l'administration par injection de liposomes vides pour saturer les organes réticuloendothéliaux pour réduire la capture par la rate et le foie non spécifique à la tumeur dudit radionucléide.

11. Utilisation selon l'une quelconque des revendications 1 à 10, dans laquelle des molécules auxiliaires sont incluses dans la formulation de liposome pour empêcher la capture par les organes réticuloendothéliaux.

12. Utilisation selon la revendication 11, dans laquelle les molécules auxiliaires sont des lipides liés à du polyéthylèneglycol (PEG-lipides).

13. Utilisation selon la revendication 6, dans laquelle les molécules de ciblage sont reliées à des molécules auxiliaires.

14. Utilisation selon l'une quelconque des revendications 1 à 13, dans laquelle des molécules supplémentaires sont incorporées dans la formulation pour faciliter la fusion membranaire avec les cellules cibles ou pour faciliter l'endocytose par les cellules cibles.
